(19) Europäisches Patentamt
European Patent Office
Office européen des brevets

(11) **EP 4 752 532 A1**

(12) **EUROPEAN PATENT APPLICATION**

(43) Date of publication:
03.06.2026 Bulletin 2026/23

(21) Application number: 25208877.8

(22) Date of filing: 15.10.2025

(51) International Patent Classification (IPC):
**G01N 21/27** (2006.01) **G01N 21/64** (2006.01)

(52) Cooperative Patent Classification (CPC):
G01N 21/6458; G01N 21/274; G01N 21/648;
G01N 33/54346; G01N 33/582

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 27.11.2024 JP 2024205926

(71) Applicant: Canon Kabushiki Kaisha
Tokyo, 146-8501 (JP)

(72) Inventors:
• EGUCHI, Akira
Tokyo, 146-8501 (JP)
• YAMAUCHI, Fumio
Tokyo, 146-8501 (JP)
• SUGAWA, Yoshihiko
Tokyo, 146-8501 (JP)

(74) Representative: Canon Europe Limited
European Intellectual Property Group
4 Roundwood Avenue
Stockley Park
Uxbridge UB11 1AF (GB)

(54) **MEASUREMENT METHOD, MEASUREMENT APPARATUS, AND PROGRAM**

(57) A measurement method includes measuring fluorescent light emitted from fluorescent dyes of each of a plurality of fine particles that are bound with the fluorescent dyes, and measuring reference light emitted from each of the plurality of fine particles, using a plurality of solutions in which the plurality of fine particles are dispersed, acquiring, for each of the plurality of solutions, a ratio of particles for which both the fluorescent light from the fluorescent dyes and the reference light are detected in the plurality of fine particles, and acquiring a minimum number of fluorescent dyes detectable by a measurement apparatus (1000), based on the ratio for each of the plurality of solutions.

FIG. 4

**Description**

TECHNICAL FIELD

The present disclosure relates to a measurement method, a measurement apparatus, and a program.

BACKGROUND

Developments of measurement technologies for measuring each microscopic specimen such as a virus and an extracellular vesicle and analyzing protein expressed in the individual specimen are underway. Each of PCT Domestic Publication No. 2023-521872 and Jeong, Mi Ho, et al. "Plasmon-Enhanced Single Extracellular Vesicle Analysis for Cholangiocarcinoma Diagnosis." Advanced Science, Vol. 10, Issue 8, pp 2205148, January 2023, USA discloses a method for measuring a single extracellular vesicle by immobilizing extracellular vesicles on a plasmon substrate using an affinity ligand selectively bound to the extracellular vesicle, and by measuring fluorescent light amplified by plasmon resonance.

SUMMARY

The present disclosure in its first aspect provides a measurement method as specified in claims 1 to 16.
The present disclosure in its second aspect provides a measurement apparatus as specified in claim 17.
The present disclosure in its third aspect provides a program as specified in claim 18.
Features of the disclosure will become apparent from the following description of embodiments with reference to the attached drawings. The following description of embodiments will be provided by way of example.

BRIEF DESCRIPTION OF THE DRAWINGS

Fig. 1 is a schematic diagram of a measurement apparatus according to a first embodiment.
Figs. 2A, 2B, 2C, and 2D are schematic diagrams of calibration particles in each embodiment.
Fig. 3 is a schematic diagram illustrating a method for calculating a detection rate of calibration particles in each embodiment.
Fig. 4 is a schematic diagram illustrating a method for calculating a detection limit according to each embodiment.
Fig. 5 is a flowchart illustrating a measurement method according to each embodiment.
Fig. 6 is a schematic diagram illustrating a measurement method according to a variation of each embodiment.
Fig. 7 illustrates an evaluation result in the first embodiment.
Fig. 8 is a schematic diagram of a measurement apparatus according to a second embodiment.

DESCRIPTION OF THE EMBODIMENTS

In the following, the term "unit" may refer to a software context, a hardware context, or a combination of software and hardware contexts. In the software context, the term "unit" refers to a functionality, an application, a software module, a function, a routine, a set of instructions, or a program that can be executed by a programmable processor such as a microprocessor, a central processing unit (CPU), or a specially designed programmable device or controller. A memory contains instructions or programs that, when executed by the CPU, cause the CPU to perform operations corresponding to units or functions. In the hardware context, the term "unit" refers to a hardware element, a circuit, an assembly, a physical structure, a system, a module, or a subsystem. Depending on the specific embodiment, the term "unit" may include mechanical, optical, or electrical components, or any combination of them. The term "unit" may include active (e.g., transistors) or passive (e.g., capacitor) components. The term "unit" may include semiconductor devices having a substrate and other layers of materials having various concentrations of conductivity. It may include a CPU or a programmable processor that can execute a program stored in a memory to perform specified functions. The term "unit" may include logic elements (e.g., AND, OR) implemented by transistor circuits or any other switching circuits. In the combination of software and hardware contexts, the term "unit" or "circuit" refers to any combination of the software and hardware contexts as described above. In addition, the term "element," "assembly," "component," or "device" may also refer to "circuit" with or without integration with packaging materials.
Referring now to the accompanying drawings, a detailed description will be given of embodiments according to the disclosure. Each of the embodiments of the present disclosure described below can be implemented solely or as a combination of a plurality of the embodiments or features thereof where necessary or where the combination of elements or features from individual embodiments in a single embodiment is beneficial.

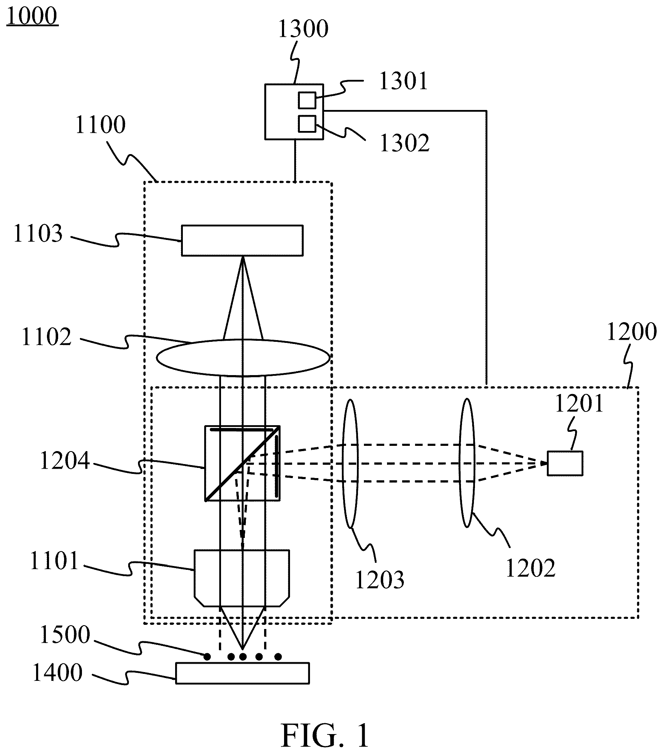

Fig. 1 is a schematic view illustrating the configuration of a fluorescence measurement apparatus 1000 according to each embodiment. The fluorescence measurement apparatus 1000 includes a microscope unit (detector) 1100, an illumination unit 1200, and a control unit 1300. A measurement unit includes the microscope unit 1100 and the illumination unit 1200. As described later, the measurement unit measures fluorescent light emitted from fluorescent dyes of each of a plurality of fine particles (or microparticles) that are labeled with the fluorescent dyes (fluorochromes) and emit reference light, and the reference light emitted from each of the plurality of fine particles, using a plurality of solutions in which the plurality of fine particles are dispersed. The fluorescence measurement apparatus 1000 irradiates a sample 1500 disposed on a substrate 1400 (chemically bound to the substrate) with illumination light and detects (measures) fluorescent light emitted from the sample 1500.

The microscope unit 1100 includes an optical system (measurement optical system) including an objective lens 1101 and an imaging lens 1102, and an image sensor 1103 such as a CMOS sensor. An enlarged image of the sample 1500 formed by the objective lens 1101 and the imaging lens 1102 is imaged by the image sensor 1103. To obtain images at different magnifications, the objective lens 1101 may be mounted on a revolver on which a plurality of objective lenses 1101 can be installed.

The illumination unit 1200 includes a light source 1201 and an illumination optical system. The illumination optical system includes a collimator lens 1202, a condenser lens 1203, and a filter cube 1204.

In the configuration illustrated in Fig. 1, the objective lens 1101 is shared by the microscope unit 1100 and the illumination unit 1200, and the illumination unit 1200 illuminates the sample 1500 with a configuration including the objective lens 1101.

The light source 1201 includes, for example, a light-emitting diode (LED) or a laser beam source but is not limited to this example. The light source 1201 may be constituted, for example, by combining a light source having a broad wavelength band, such as a halogen lamp or a white LED, with a proper band-pass filter. The illumination unit 1200 constitutes a Kohler illumination system using the collimator lens 1202, the condenser lens 1203, and the objective lens 1101.

The filter cube 1204 has a wavelength characteristic that reflects illumination light and transmits fluorescent light emitted from the sample 1500. The filter cube having such a wavelength characteristic may be configured, for example, by a combination of a band-pass filter that transmits only illumination light, a dichroic mirror that reflects only illumination light and transmits fluorescent light from the sample, and a band-pass filter that transmits only fluorescent light. As a simpler configuration, the filter cube 1204 may have a configuration combining a single band-pass filter and a dichroic mirror, or a configuration constituted by only a dichroic mirror. A plurality of filter cubes may be provided and switched in accordance with a target fluorescent dye. In this case, to facilitate switching, the filter cubes may be installed on a filter wheel that allows for selection of a filter cube to be used from among a plurality of filter cubes.

The control unit 1300 includes a dedicated computer or a personal computer and controls lighting of the light source of the illumination unit 1200, driving of an unillustrated drive mechanism, and image acquisition by the microscope unit 1100 in accordance with computer programs. More specifically, the control unit 1300 switches an illumination wavelength and the filter cube 1204 by communicating with the illumination unit 1200, and acquires a fluorescence image at each wavelength by communicating with the microscope unit 1100. The control unit 1300 may perform calculations to estimate the detection limit, which will be described later.

The control unit 1300 and each component may be directly connected to each other through a cable or the like, or may be connected to each other by using a short-distance communication system. The control unit 1300 may have functions to store images, perform calculations using images, display images, and the like, in addition to controlling the microscope unit 1100 and the illumination unit 1200. These functions may be achieved by another device through a network. Analyzing acquired images can provide information on proteins, RNA, and the like contained in the sample 1500.

The control unit 1300 includes a first acquiring unit 1301 and a second acquiring unit 1302. The first acquiring unit 1301 acquires, for each of a plurality of solutions, a ratio of particles (detection rate $r_i$) for which both the fluorescent light from the fluorescent dyes and the reference light are detected among a plurality of fine particles. The second acquiring unit 1302 acquires the minimum number of fluorescent dyes (detection limit $x_{min}$) detectable by the fluorescence measurement apparatus 1000 based on the ratios for each of the plurality of solutions.

The substrate 1400 is an element for enabling microscope observation of the sample 1500 immobilized on its surface and is, for example, a glass substrate, a substrate with a surface coated with dielectric or metal, or a plasmon substrate that causes plasmon resonance with a fine metal structure, but it is not limited to this example. A ligand (material specifically bound to a particular receptor) that selectively binds the sample 1500 onto the substrate may be provided on the surface of the substrate 1400.

The sample 1500 is a measurement target object and varies according to the purpose of measurement. In the evaluation and calibration of the measurement apparatus, fluorescent particles for calibration to be described later serve as the sample 1500. After the calibration is performed, for example, extracellular vesicles (such as exosomes) derived from biological tissue, viruses, or liposomes serve as the sample 1500. In each embodiment, fluorescent particles to be used as the sample 1500 during calibration will be referred to as calibration particles, and the sample 1500 to be measured after calibration will be referred to as a specimen.

In the specimen, the fluorescent dye is bound via an antibody corresponding to a target protein. When a fluorescence image of the specimen is acquired by the fluorescence measurement apparatus 1000, a plurality of light emission points are observed in the image in a case where the specimen contains the target protein. Since each light emission point is generated by fluorescent light emitted from an individual specimen containing the target protein, the amount of the specimen containing the target protein can be evaluated by evaluating the number of light emission points (the number of detections, detection count) in the image.

Such bright spots cannot be detected by the fluorescence measurement apparatus 1000 unless a certain number of fluorescent dyes are bound to the specimen. Thus, it is important to evaluate the minimum number of fluorescent dyes per specimen for detection by the fluorescence measurement apparatus 1000, i.e., the detection limit for the number of fluorescent dyes. One conceivable method for evaluating the detection limit of the fluorescence measurement apparatus 1000 for the number of dyes is to evaluate whether the particles can be detected, using the calibration particles in which the binding number of fluorescent dyes have been previously checked.

Since fluorescent light emitted from a microscopic specimen such as an extracellular vesicle or virus, which is several tens to several hundreds of nanometers is weak, a highly sensitive fluorescence measurement method is demanded. In order to improve the sensitivity, one conceivable method is to immobilize a specimen on a plasmon substrate or a metal-coated substrate, and observe it as disclosed in PCT Domestic Publication No. 2023-521872. However, the light emission intensity varies on such a substrate according to the size and refractive index of the specimen and the dye binding method. That is, the detection limit can vary according to the specimen.

Therefore, the calibration may use fluorescent particles that mimic the size, refractive index, and dye binding method of a specimen to be measured. In this case, calibration particles of tens to hundreds of nanometers in size may be prepared, and even the fluorescent light from the calibration particles will be weak. As a result, the detection limit of the fluorescence measurement apparatus 1000 cannot be accurately evaluated.

In order to solve these problems, each embodiment uses, as calibration particles, particles having a size comparable to that of the specimen, to which evaluation fluorescent dyes are bound and emitting sufficiently bright reference light.

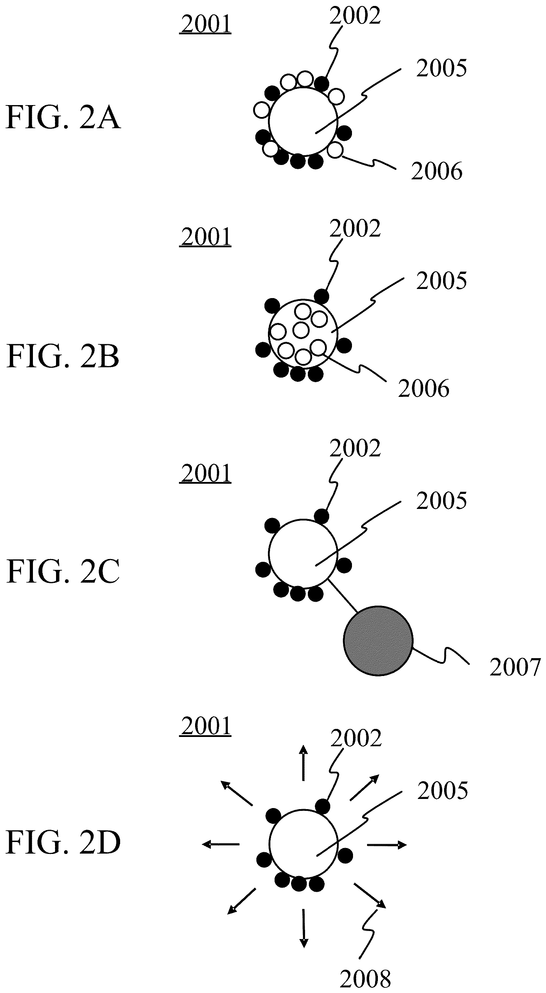

The calibration particles (plurality of microscopic or tiny particles) 2001 will be described with reference to Figs. 2A, 2B, 2C, and 2D. Figs. 2A, 2B, 2C, and 2D are schematic diagrams of the calibration particles 2001 according to each embodiment. The material of a particle 2005 to which fluorescent dyes are bound can use general particle material, such as silica or polystyrene. Since a biologically derived substance has a small refractive index, silica may be used as the material. The size of the calibration particles 2001 may be equivalent to that of the specimen. In a case where, for example, extracellular vesicles or viruses are assumed as the specimen, the diameter of the particle 2005 is desirably 10 nm to 500 nm, or 40 nm to 200 nm.

The evaluation fluorescent dye 2002 is a fluorescent dye with which the fluorescence measurement apparatus 1000 is calibrated. The evaluation fluorescent dye 2002 may be the same as a fluorescent dye that is used when the specimen is measured. The fluorescent dye 2002 and the particle 2005 can be bound by general chemical reaction such as amide bonding or antibody reaction, but a method that is the same method for binding the specimen and the evaluation fluorescent dye 2002 may be used. For example, in a case where an evaluation fluorescent dye is bound to the specimen via an antibody, the particle 2005 and the evaluation fluorescent dye 2002 may be bound via the antibody as well. In a case where the specimen and the fluorescent dye 2002 are bound via a plurality of antibodies, the particle 2005 and the evaluation fluorescent dye 2002 may be bound via the plurality of antibodies in a similar manner. In a case where the target protein in the specimen is a protein expressed on a membrane of the specimen, the evaluation fluorescent dye 2002 may be bound on the surface of the particle 2005. In a case where the target protein is a protein expressed inside the specimen, the evaluation fluorescent dye 2002 may be encapsulated in the particle 2005.

Light for reference (reference light) is fluorescent light from a fluorescent dye having absorption and emission wavelengths different from those of the evaluation fluorescent dye 2002. For example, the wavelength of the reference light is shorter than that of light from the fluorescent dye. The fluorescent light emitted from an evaluation fluorescent dye can be prevented from being absorbed by the reference fluorescent dye. However, each embodiment is not limited to this example. Each example excellently functions by binding a fluorescent dye having such a wavelength characteristic to the particle 2005 as a reference fluorescent dye 2006 as illustrated in Fig. 2A. Not only fluorescent dyes but also light emission materials such as quantum dots may be bound to the particle.

As illustrated in Fig. 2B, the reference fluorescent dye 2006 may be encapsulated in the particle 2005. Encapsulating the reference fluorescent dye 2006 in the particle 2005 can have a larger number of dyes than those bound to the surface, thereby providing more reference fluorescence. As illustrated in Fig. 2C, a metal nanoparticle 2007 may be bound to the particle 2005. The metal nanoparticle 2007 emits strong scattered light, and thus the scattered light from the metal nanoparticle 2007 can be used as the reference light. As illustrated in Fig. 2D, scattered light 2008 emitted from the particle 2005 may be used as light for reference (reference light). The reference light is used to specify the positions and number of calibration particles as described later, and thus any light that allows the specification can be used as the reference light. The following description will be made with the calibration particle illustrated in Fig. 2A as an example.

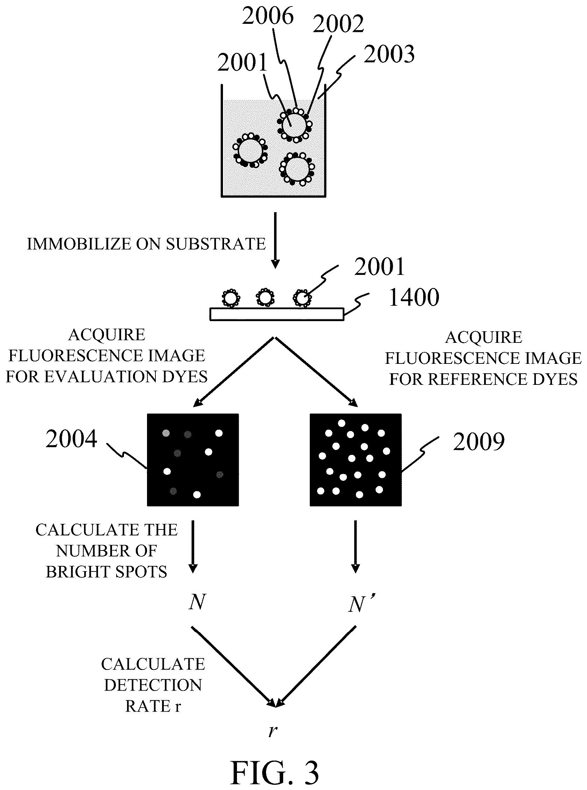

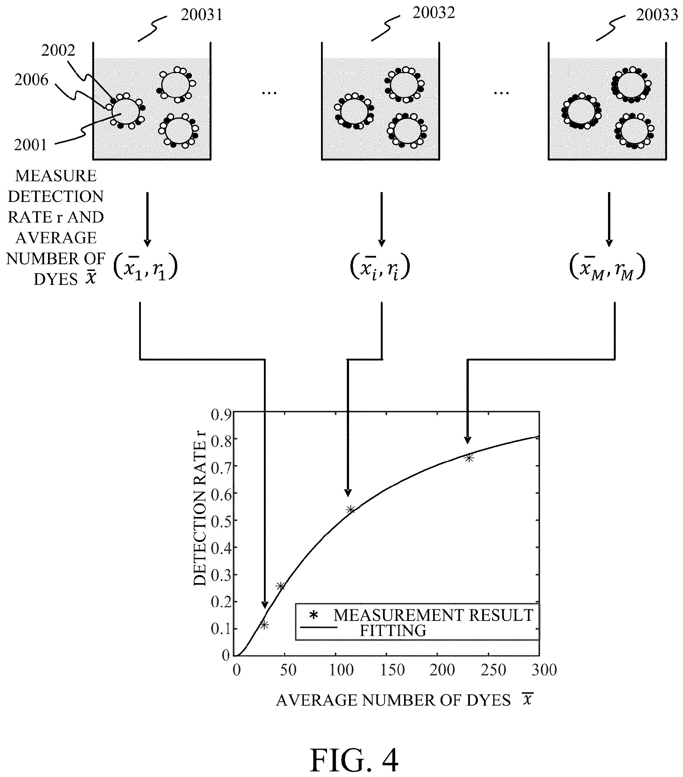

Referring now to Figs. 3 and 4, a description will be given of a method for evaluating the detection limit of the fluorescence measurement apparatus 1000 for the number of dyes using calibration particles 2001. First, a method for calculating the detection rate of the calibration particles 2001 will be described with reference to Fig. 3. Fig. 3 is a schematic diagram illustrating a method for calculating the detection rate of the calibration particles 2001.

The solution 2003 in which the calibration particles 2001 are dispersed is dropped onto the substrate 1400, and the calibration particles 2001 are immobilized on the substrate 1400. The immobilizing method is not particularly limited but may be the same as a method for immobilizing the specimen onto the substrate. For example, in a case where the specimen and the substrate are chemically bound by via a ligand, the calibration particles may be immobilized to the substrate using the same ligand. In a case where a solution containing the specimen is dried for immobilization, the calibration particles 2001 may be immobilized by drying in a similar manner.

The control unit 1300 in the fluorescence measurement apparatus 1000 acquires fluorescence images of the immobilized fluorescent particles. The fluorescence images are two captured images: a fluorescence image (first image) 2004 for the evaluation fluorescent dye 2002 and a fluorescence image (second image) 2009 for the reference fluorescent dye 2006 (the reference light). The measurement order is not limited, and the measurement may be simultaneously performed.

In each image, bright spots having a size determined by the objective lens 1101 are observed at a plurality of places. Since evaluation fluorescent light and reference fluorescent light are emitted from the same particle, the bright spots exist at substantially the same positions in the two fluorescence images 2004 and 2009. These bright spots are detected from the two images through image processing. The number N of bright spots (bright spot number N) of the evaluation dyes (first bright spot number of bright spots included in the fluorescence image 2004) are acquired (extracted) from the evaluation fluorescence image 2004. The number N' of bright spots (bright spot number N') (second bright spot number of spots included in the fluorescence image 2009) is acquired (extracted) from the reference fluorescence image 2009.

The method for extracting bright spots is not particularly limited and can be implemented by a general image processing method. Since a reference dye is sufficiently bright, the number of bright spots included in the reference fluorescence image 2009 indicates the number of particles existing in the image. Bright spots included in the evaluation fluorescence image 2004 indicate particles that emit fluorescent light beyond the detection limit of the fluorescence measurement apparatus 1000. Thus, as expressed by equation (1) below, the ratio of detected particles (the detection rate r) is calculated using a ratio of the bright spot number N to the bright spot number N':

$$r = \frac{N}{N'} \qquad\qquad (1)$$

Referring now to Fig. 4, a description will be given of a method for calculating the detection limit for the number of dyes from the detection rate r. Fig. 4 is a schematic diagram illustrating the method for calculating the detection limit.

Each embodiment utilizes a plurality of types of solutions (particles dispersed solutions) in which the calibration particles 2001 are dispersed. The plurality of solutions (dispersed solutions) 20031, 20032, and 20033 differ from one another in the number of evaluation fluorescent dyes 2002 bound to the calibration particles 2001 in each solution. The solutions 20031, 20032, and 20033 illustrated in Fig. 4 have increasing numbers of evaluation fluorescent dyes 2002 in order of their reference numerals. The control unit 1300 acquires a detection rate $r_i$ for each of the plurality of solutions 20031, 20032, and 20033. Here, i represents the solution number and takes values from 1 to M, where M is the number of solutions. For convenience, the number of evaluation fluorescent dyes 2002 increases in order of the i number.

On the other hand, the average number of fluorescent dyes 2002 bound per particle (the average number of dyes $x_i$) $\overline{x_i}$ is measured for each solution. The average number of dyes $\overline{x_i}$ can be calculated from the measurement result of the solution absorbance and particle number concentration. Since the fluorescent particles are typically dispersed in the solution, the absorbance A of the solution 2003 (20031-20033) can be measured. This measurement may use a general absorptiometer. The absorbance A is expressed as follows using a molar absorption coefficient $\varepsilon$ of the fluorescent dyes, dye concentration $c_A$, and a thickness L of a container containing the solution:

$$A = \varepsilon c_A L \qquad\qquad (2)$$

The molar absorption coefficient $\varepsilon$ is generally known. Thus, by using equation (2), the dye concentration $c_A$ can be obtained by equation (3) below:

$$c_A = \frac{A}{\varepsilon L} \qquad (3)$$

In addition to the absorbance measurement, the number (particle number concentration) $c_p$ of particles per unit volume of the solution 2003 containing the calibration particles 2001 is measured. This can be measured by a general dynamic scattering method or a nanoparticle tracking analysis method. Alternatively, this can be calculated by comparing the absorption spectrum of a solution with a known concentration and the absorption spectrum of the solution 2003 containing the calibration particles.

By calculating a ratio of $c_A$ and $c_p$ obtained earlier, an average value $\bar{x}$ can be found from equation (4) below:

$$\bar{x} = \frac{c_A}{c_p} \qquad (4)$$

By performing this measurement for all solutions, the average number of dyes $\bar{x}_i$ for each solution can be determined.

A lower graph in Fig. 4 illustrates an evaluation result based on the dataset $(\bar{x}_i, r_i)$ of the obtained average number of dyes $\bar{x}_i$ and a plurality of detection rates $r_i$. In this graph, the horizontal axis represents the average number of dyes $\bar{x}_i$, and the vertical axis represents the detection rate r. As illustrated in Fig. 4, the detection rate r increases as the average number of dyes $\bar{x}_i$ increases.

The control unit 1300 estimates the detection limit of the fluorescence measurement apparatus 1000 for the number of dyes from the dataset $(\bar{x}_i, r_i)$. Particles dispersed in the solution are bound to randomly varying numbers of dyes. Where x is the number of fluorescent dyes bound to a single particle, the probability of a particle with x fluorescent dyes appearing in the i-th solution is considered to follow the probability density function $f_i(x)$. Since the detection rate $r_i$ is a ratio of particles in which the number of dyes x is equal to or greater than the detection limit $x_{min}$ of the fluorescence measurement apparatus 1000 (the minimum number of fluorescent dyes detectable by the fluorescence measurement apparatus 1000), the detection rate $r_i$ has the following relationship with a cumulative distribution function $F_i(x)$ of $f_i(x)$:

$$r_i = 1 - F_i(x_{min}) \qquad (5)$$

Since the number of dyes to be bound is random and nonnegative, a log-normal distribution function can be used as a good example of the probability density function $f_i(x)$. That is, $f_i(x)$ can be expressed as illustrated in the following equation (6):

$$f_i(x) = \frac{1}{\sqrt{2\pi\sigma^2}x} \exp\left(-\frac{(\ln x - \mu_i)^2}{2\sigma^2}\right) \qquad (6)$$

$\mu_i$ and $\sigma$ are coefficients that determine the log-normal distribution function (coefficients associated with the detection rate $r_i$). Due to the characteristics of the log-normal distribution, $\sigma$ corresponds to a ratio of the average value to the standard deviation and is considered to be the same between the solutions in a range where the dye concentration does not vary significantly. On the other hand, $\mu$ has a value corresponding to the average value of the log-normal distribution, i.e., the average number of bound dyes $\bar{x}_i$ and is therefore an amount that varies between the solutions.

Using the cumulative distribution function for the log-normal distribution, equation (5) can be expressed as equation (7) below:

$$r_i = \frac{1}{2} - \frac{1}{2}\operatorname{erf}\left(\frac{\ln x_{min} - \mu_i}{\sigma\sqrt{2}}\right) \qquad (7)$$

where erf is an error function.

Based on the characteristics of the log-normal distribution, the average number of dyes $\bar{x}_i$ can be expressed as in equation (8) using the coefficients $\mu_i$ and $\sigma$.

$$\bar{x}_i = e^{\mu_i + \frac{\sigma^2}{2}} \qquad (8)$$

Solving equation (8) for the coefficient $\mu_i$ and substituting it into equation (7) yields equation (9):

$$r_i = \frac{1}{2} - \frac{1}{2}\operatorname{erf}\left(\frac{\ln x_{min} - \ln \bar{x}_i}{\sigma\sqrt{2}} + \frac{\sigma}{2\sqrt{2}}\right) \qquad (9)$$

Equation (9) is a function that represents a relationship of the data set ($\overline{x}_i$, $r_i$). Using this function, $x_{min}$ and $\sigma$ that best reproduce the data set ($\overline{x}_i$, $r_i$) as a measurement result can be determined by fitting, etc., to find $x_{min}$. For example, the fitting result can be obtained as illustrated by a solid line in Fig. 4.

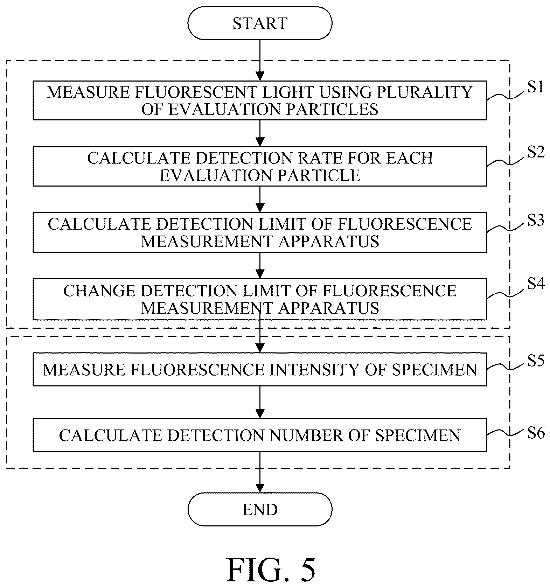

Referring now to Fig. 5, a description will be given of a calibration method (measurement method) for the fluorescence measurement apparatus 1000 according to each embodiment. Fig. 5 is a flowchart illustrating the measurement method.

First, in step S1, the control unit 1300 in the fluorescence measurement apparatus 1000 acquires a fluorescence image using calibration particles (measures fluorescent light using a plurality of calibration particles). That is, the calibration particles are immobilized on the substrate 1400, and the control unit 1300 acquires fluorescence images 2004 and 2009 using a light source of a wavelength corresponding to each fluorescent dye, and a fluorescence filter.

Next, in step S2, the control unit 1300 performs image processing for the acquired fluorescence images 2004 and 2009, acquires (calculates) the number of bright spots $N_i$ and $N_i'$ in each fluorescence image, and acquires (calculates) the detection rate $r_i$ using equation (1).

Next, in step S3, the control unit 1300 acquires (calculates) the detection limit $x_{min}$ for the number of dyes of the fluorescence measurement apparatus 1000 using a probability density function including the detection rate $r_i$ and the previously measured average number of dyes $\overline{x}_i$. For example, the control unit 1300 calculates $x_{min}$ and $\sigma$ that best reproduce the data set of the average number of dyes $\overline{x}_i$ and the detection rate $r_i$ by fitting using equation (9).

Next, in step S4, the control unit 1300 changes (updates) the value of the detection limit $x_{min}$ for the number of dyes recorded in the fluorescence measurement apparatus 1000 to the value obtained by the processing of steps S1 to S4. The value may be changed by a user inputting the calculation result into the fluorescence measurement apparatus 1000 via an input device, or the fluorescence measurement apparatus 1000 may automatically update it.

Steps S1 to S4 are calibration steps for calibrating the fluorescence measurement apparatus 1000. Since the sensitivity of the fluorescence measurement apparatus 1000 changes due to the deterioration over time and changes in the external environment, the calibration step may be performed periodically or for each measurement.

Next, in step S5, the control unit 1300 measures the fluorescent light of the specimen. That is, the control unit 1300 immobilizes the specimen labeled with a fluorescent dye for evaluation on the substrate 1400 and acquires a fluorescence image.

Next, in step S6, the control unit 1300 performs image processing for the fluorescence image obtained in step S5, and acquires (calculates) the number of bright points in the fluorescence image, i.e., the number of detections.

The above method allows for highly accurate measurement of the detection limit $x_{min}$ for the number of dyes of the fluorescence measurement apparatus 1000. Evaluating the detection limit $x_{min}$ over time can not only ascertain the deterioration status and maintenance schedule of the fluorescence measurement apparatus 1000, but also evaluate the superiority or inferiority of fluorescence measurement apparatus by comparing the detection limits $x_{min}$ between fluorescence measurement apparatuses. The detection limit for the number of dyes means that if a single specimen contains the number of dyes equal to or greater than the detection limit $x_{min}$, the specimen can be detected from the fluorescence image 2004. Finding the detection limit for the number of dyes ensures that the specimen detected by the fluorescence measurement apparatus 1000 contains at least the number of fluorescent dyes equal to or greater than the detection limit $x_{min}$.

The known number of dyes $\beta$ per ligand, such as an antibody that is used to bind dyes to the specimen, can be used. By using the number of dyes $\beta$, the minimum amount of protein per specimen for detection of the fluorescence measurement apparatus 1000 (minimum measurable amount), i.e., the detection limit $y_{min}$ for the amount of protein, can be obtained (calculated), as expressed in the following equation (10):

$$y_{min} = \frac{x_{min}}{\beta} \qquad\qquad (10)$$

In other words, the detection limit of the protein amount means that if a single specimen expresses proteins equal to or greater than the detection limit $y_{min}$ of the protein amount, then this specimen can be detected from the fluorescence image 2004. Evaluating the detection limit $y_{min}$ before the specimen is measured can ensure that the detected specimen expresses proteins equal to or greater than the detection limit $y_{min}$.

In each embodiment, the value calculated and updated in the calibration step is not limited to the detection limit $x_{min}$ for the number of dyes, but may also be the detection limit $y_{min}$ for the number of proteins.

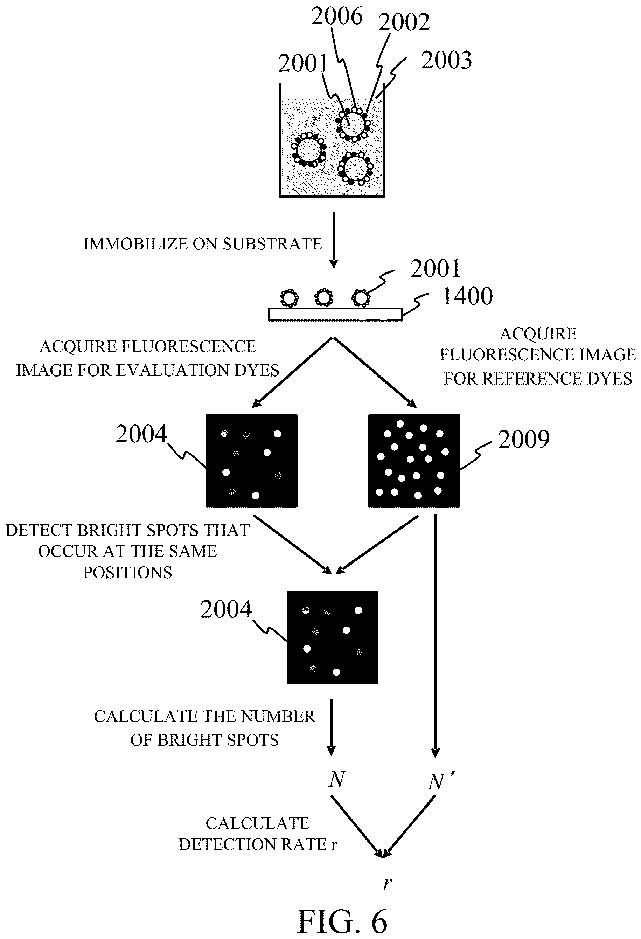

Referring now to Fig. 6, a description will be given of a calibration method (measurement method) according to a variation of each embodiment. Fig. 6 is a schematic diagram illustrating the measurement method according to the variation. As illustrated in Fig. 6, in detecting bright spots from the fluorescence image 2004, the two fluorescence images 2004 and 2009 may be compared to identify bright spots occurring at approximately the same positions.

Since the light from the evaluation dye is weak, unexpected light emission from impurities or noise generated by the image sensor may be mixed into the fluorescence image 2004. Since such impurities or noise are rarely observed at approximately the same positions in two images, unnecessary signals can be removed by identifying only the bright spots that occur at the same locations in the two images. Here, "approximately the same positions" do not mean the exact same position, and a range to some extent, such as a few times the size of the point spread function of the objective lens 1101 or within a few pixels, can be considered to be the same position. Calculating the number N of bright spots identified as being at the same position and the detection limit using the above method can provide highly accurate calibration with reduced influence of unnecessary signals.

Each embodiment has discussed the log-normal distribution function as the probability density function, but is not limited to this example. Functions similar to the log-normal distribution include a gamma distribution, a beta distribution, and a Weibull distribution, and they may also be used as the probability density function f(x) for analysis. In any of the functions, a plurality of coefficients representing the probability density function are associated by the equation representing the detection rate expressed by equation (5) and the average number of dyes, which corresponds to equation (8). The detection limit $x_{min}$ of the fluorescence measurement apparatus 1000 can be calculated by analyzing the data set of the detection rate and the average number of dyes using fitting or another method based on these relational equations. The log-normal distribution may be set as the probability density function because it accurately represents the frequency of the number of dyes $x_i$ and is easy to handle.

Each embodiment has discussed the method of calculating the coefficients of the probability density function using fitting, but is not limited to this example. $\sigma$ in the log-normal distribution is a value that is generally determined according to the particle. $\sigma$ can be calculated by performing the above method using a fluorescence measurement apparatus other than the fluorescence measurement apparatus 1000 to be evaluated, and then solving equation (9) to calculate the detection limit $x_{min}$ without using fitting.

Each embodiment can also be used to evaluate calibration particles. Different calibration particles can be measured using the same fluorescence measurement apparatus 1000, and the detection limit $x_{min}$ values can be compared between particles to evaluate the superiority or inferiority. Each specific embodiment will be described in detail below.

FIRST EMBODIMENT

A first embodiment will now be described. The fluorescence measurement apparatus 1000 according to this embodiment, illustrated in Fig. 1, includes two light sources 1201, an LED with a center wavelength of 475 nm and an LED with a center wavelength of 630 nm.

As filter cubes (fluorescence filters) 1204, a fluorescence filter set 1 for observing green fluorescent dyes and a fluorescence filter set 2 for observing red fluorescent dyes are provided to the turret. The fluorescence filter set 1 includes an excitation filter with a center wavelength of 480 nm and a bandwidth of 30 nm, a long-pass dichroic mirror with a cut-on wavelength of 505 nm, and an absorption filter with a center wavelength of 535 nm and a bandwidth of 40 nm. The fluorescence filter set 2 includes an excitation filter with a center wavelength of 620 nm and a bandwidth of 50 nm, a long-pass dichroic mirror with a cut-on wavelength of 655 nm, and an absorption filter with a center wavelength of 690 nm and a bandwidth of 50 nm.

The illumination unit 1200 includes a general collimator lens 1202 and condenser lens 1203, and together with the objective lens 1101, constitutes a Köhler illumination system. The objective lens 1101 has a magnification of 40x and an NA of 0.95. A fluorescence image of the sample 1500 immobilized on the substrate 1400 is formed on the image sensor (CMOS sensor) 1103 via the objective lens 1101, filter cube 1204, and imaging lens 1102.

The calibration particles are created by binding silica particles to a cyanine-based red fluorescent dye with an NHS ester at its end as the evaluation fluorescent dye, and a fluorescein-based green fluorescent dye with an NHS ester at its end as the reference fluorescent dye. The silica particles are approximately 100 nm in diameter and have amino groups on their surfaces. A sufficient amount of the reference green fluorescent dye is bound to the silica particles. Five types of calibration particles are created, each with a different amount of red fluorescent dye bound to it for evaluation. These particle dispersion solutions are dropped onto a glass substrate and a gold-coated Si substrate, followed by drying, to immobilize the calibration particles. Fluorescence images of the immobilized particles are acquired using a light source wavelength and fluorescence filters corresponding to the two fluorescent dyes.

Bright spots are extracted from the obtained fluorescence images through image processing, and the detection rate $r_i$ (i = 1 to 5) for each solution is calculated. The average dye number $\overline{x_i}$ (i = 1 to 5) for each solution is calculated by measuring the absorbance and particle number concentration for each particle solution.

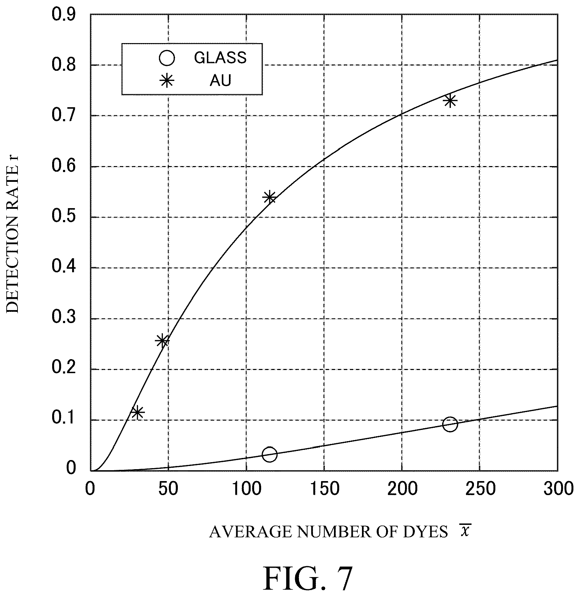

Fig. 7 illustrates the obtained dataset of the detection rate $r_i$ and average dye number $\overline{x_i}$. In Fig. 7, the horizontal axis represents the average dye number $\overline{x}$, and the vertical axis represents the detection rate r. Furthermore, a log-normal distribution as the probability density function is assumed, $x_{min}$ and $\sigma$ that best reproduce the relationship between the detection rate $r_i$ and the average dye number $\overline{x}$ are calculated by fitting using equation (9). Obtained $x_{min}$ may be, for example, 561 in a case where glass is used and 53 in a case where a gold-deposited substrate is used. Thus, $x_{min}$ can be calculated. The detection limit is smaller for the gold-deposited substrate than for the glass substrate. Thus, it is possible not only to calculate the detection limit but also to compare substrates and devices.

Assume that the number of dyes bound to a single ligand β is typically 5. Then, due to equation (10), the detection limit $y_{min}$ for the protein amount in the fluorescence measurement apparatus 1000 can be calculated as 112 in a case where a glass substrate is used and 11 in a case where a gold-deposited substrate is used.

SECOND EMBODIMENT

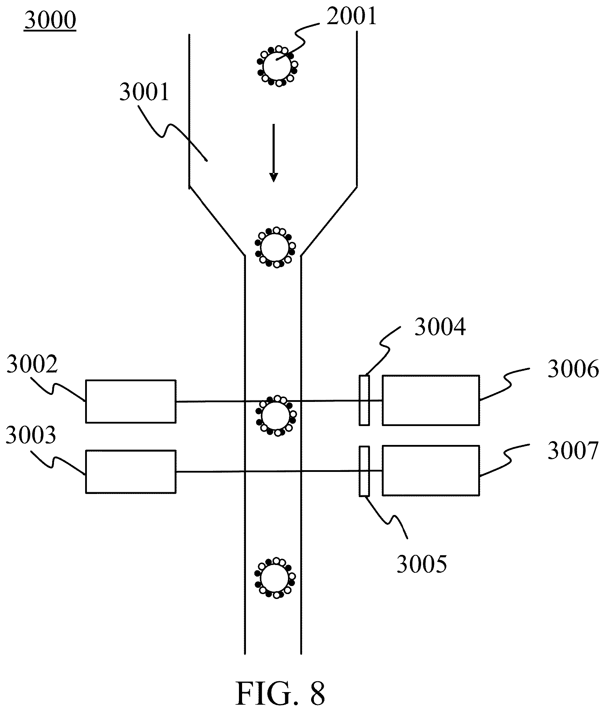

Next, a second embodiment will be described. This embodiment uses a flow cytometer 3000 as a fluorescence measurement apparatus. Fig. 8 is a schematic diagram of the flow cytometer (measurement apparatus) 3000 according to this embodiment.

The flow cytometer 3000 irradiates light from laser light sources 3002 and 3003 onto the calibration particles 2001 flowing through a flow path 3001. Fluorescent light from the calibration particles 2001 is detected by photomultiplier tubes 3006 and 3007 through bandpass filters 3004 and 3005. The flow path 3001 has a narrow flow path that allows a single particle to flow through the laser irradiation area. The laser light source 3002 is a semiconductor laser with a wavelength of 488 nm. The laser light source 3003 is a semiconductor laser with a wavelength of 635 nm. The bandpass filter 3004 has a center wavelength of 525 nm and a bandwidth of 50 nm. The bandpass filter 3005 has a center wavelength of 700 nm and a bandwidth of 50 nm.

The calibration particles are the same as those in the first embodiment. The calibration particles are flowed at a flow rate that allows the fluorescent light of the evaluation dye and the signal of the reference dye to be determined to be emitted from the same particle in terms of time, and the fluorescence intensity (fluorescent light intensity) of each is measured. In this embodiment, the current values output from photomultiplier tubes 3006 and 3007 represent the fluorescence intensity. Let N' be the number of observed reference fluorescent light (second number of bright spots) and N be the number of measured evaluation fluorescent light (first number of bright spots). Based on equation (1), the detection rate $r_i$ is calculated for each particle. The detection limit is calculated by the calculation similar to that of the first embodiment using the data set of the detection rate $r_i$ and the average number of dyes $\bar{x}$.

The flow cytometer 3000 may include a photomultiplier tube that measures side scattering. In a case where the particles illustrated in Fig. 2C or 2D are used as the calibration particles 2001, the side scattered light can be used as the reference light. The wavelength characteristics of the laser light sources 3002 and 3003 and the bandpass filters 3004 and 3005 are properly changed according to the fluorescent dye to be evaluated.

OTHER EMBODIMENTS

Embodiment(s) of the disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)TM), a flash memory device, a memory card, and the like.

While the disclosure has described example embodiments, it is to be understood that the disclosure is not limited to the example embodiments. The scope of the following claims is to be accorded the broadest interpretation so as to encompass all such modifications and equivalent structures and functions.

Each embodiment according to the disclosure can provide a measurement method that can evaluate the detection limit of a single specimen in a measurement apparatus.

**Claims**

**1.** A measurement method comprising:

measuring (S1) fluorescent light emitted from fluorescent dyes (2002) of each of a plurality of fine particles (2001) that are bound with the fluorescent dyes (2002), and measuring (S1) reference light emitted from each of the plurality of fine particles (2001), using a plurality of solutions (20031, 20032, 20033) in which the plurality of fine particles (2001) are dispersed;

acquiring, for each of the plurality of solutions (20031, 20032, 20033), a ratio (r) of particles (2001) for which both the fluorescent light from the fluorescent dyes (2002) and the reference light are detected in the plurality of fine particles; and

acquiring (S3) a minimum number of fluorescent dyes (2002) detectable by a measurement apparatus (1000), based on the ratio for each of the plurality of solutions (20031, 20032, 20033).

2. The measurement method according to claim 1, **characterized in that** the average value of the number of fluorescent dyes (2002) bound to the fine particles (2001) is different for each of the plurality of solutions (20031, 20032, 20033).

3. The measurement method according to claim 1 or 2, **characterized in that** acquiring the minimum number acquires the minimum number using a function having the ratio (r).

4. The measurement method according to claim 3, **characterized in that** the function is a log-normal distribution function having a coefficient associated with the ratio.

5. The measurement method according to claim 3 or 4, **characterized in that** the function is expressed as:

$$r_i = 1 - F_i(x_{min})$$

where $r_i$ is the ratio of an i-th solution among the plurality of solutions, $x_{min}$ is the minimum number, and $F_i(x)$ is a cumulative distribution function of a probability density function that the fine particles (2001) to which x of the fluorescent dyes (2002) are bound present in the i-th solution.

6. The measurement method according to any one of claims 1 to 5, further comprising:
updating (S4) the minimum number stored in the measurement apparatus (1000) using the minimum number.

7. The measurement method according to any one of claims 1 to 6, **characterized in that** acquiring the ratio includes:

immobilizing the plurality of fine particles (2001) on a substrate (1400),
acquiring a first image (2004) of the plurality of fine particles (2001) for the fluorescent dyes and a second image (2009) of the plurality of fine particles (2001) for the reference light,
acquiring a first number of bright spots (N) located at the same position in the first image and the second image and a second number of bright spots (N') included in the second image, and
acquiring the ratio (r) of the particles by acquiring a ratio of the first number of bright spots to the second number of bright spots.

8. The measurement method according to any one of claims 1 to 7, further comprising:
measuring fluorescent light of a specimen labeled with fluorescent dyes that are the same as the fluorescent dyes bound to the plurality of fine particles (2001).

9. The measurement method according to any one of claims 1 to 8, **characterized in that** the fine particles (2001) have diameters of 10 nm to 500 nm.

10. The measurement method according to any one of claims 1 to 9, **characterized in that** the fine particles (2001) are silica or polystyrene.

11. The measurement method according to any one of claims 1 to 10, **characterized in that** the reference light is scattered light from the fine particles (2001) or fluorescent light from fluorescent dyes (2006) different from the fluorescent dyes (2002) bound on the fine particles (2001).

12. The measurement method according to any one of claims 1 to 11, further comprising:
acquiring a minimum amount of protein measurable by the measurement apparatus (1000).

13. The measurement method according to any one of claims 1 to 12, further comprising:

acquiring the number of detections of a specimen.

14. The measurement method according to any one of claims 1 to 13, **characterized in that** a wavelength of the reference light is shorter than a wavelength of light from the fluorescent dye (2002).

15. The measurement method according to any one of claims 1 to 14, **characterized in that** the fine particles (2001) include an extracellular vesicle or a virus.

16. The measurement method according to any one of claims 1 to 15, **characterized in that** measuring the fluorescent light and the reference light includes immobilizing the fine particles (2001) on a plasmonic substrate.

17. A measurement apparatus (1000) comprising:

a measurement unit (1100, 1200) configured to measure fluorescent light from fluorescent dyes (2002) of each of a plurality of fine particles (2001) that are bound with the fluorescent dyes (2002), and configured to measure reference light emitted from each of the plurality of fine particles (2001), using a plurality of solutions (20031, 20032, 20033) in which the plurality of fine particles (2001) are dispersed;
a first acquiring unit (1301) configured to acquire, for each of the plurality of solutions (20031, 20032, 20033), a ratio of particles for which both the fluorescent light from the fluorescent dyes (2002) and the reference light are detected in the plurality of fine particles (2001), and
a second acquiring unit (1302) configured to acquire a minimum number of fluorescent dyes (2002) detectable by the measurement apparatus (1000), based on the ratio for each of the plurality of solutions (20031, 20032, 20033).

18. A program that when executed on a computer causes the computer to perform the measurement method according to any one of claims 1 to 16.

1000

1300

1301

1302

1100

1103

1102

1200

1201

1204

1203

1202

1101

1500

1400

FIG. 1

FIG. 2A

FIG. 2B

FIG. 2C

FIG. 2D

IMMOBILIZE ON SUBSTRATE

ACQUIRE
FLUORESCENCE IMAGE
FOR EVALUATION DYES

ACQUIRE
FLUORESCENCE IMAGE
FOR REFERENCE DYES

CALCULATE THE
NUMBER OF
BRIGHT SPOTS

$N$

$N'$

CALCULATE
DETECTION
RATE r

$r$

FIG. 3

FIG. 4

START

MEASURE FLUORESCENT LIGHT USING PLURALITY OF EVALUATION PARTICLES — S1

CALCULATE DETECTION RATE FOR EACH EVALUATION PARTICLE — S2

CALCULATE DETECTION LIMIT OF FLUORESCENCE MEASUREMENT APPARATUS — S3

CHANGE DETECTION LIMIT OF FLUORESCENCE MEASUREMENT APPARATUS — S4

MEASURE FLUORESCENCE INTENSITY OF SPECIMEN — S5

CALCULATE DETECTION NUMBER OF SPECIMEN — S6

END

FIG. 5

2006 2002

2001 2003

IMMOBILIZE ON SUBSTRATE

2001

1400

ACQUIRE FLUORESCENCE
IMAGE FOR EVALUATION DYES

ACQUIRE
FLUORESCENCE IMAGE
FOR REFERENCE DYES

2004

2009

DETECT BRIGHT SPOTS THAT
OCCUR AT THE SAME
POSITIONS

2004

CALCULATE THE NUMBER
OF BRIGHT SPOTS

$N$ $N'$

CALCULATE
DETECTION RATE r

$r$

FIG. 6

FIG. 7

FIG. 8

**EUROPEAN SEARCH REPORT**

Application Number

EP 25 20 8877

## DOCUMENTS CONSIDERED TO BE RELEVANT

| Category | Citation of document with indication, where appropriate, of relevant passages | Relevant to claim |
|---|---|---|
| X | US 2021/041342 A1 (TAHARA KATSUTOSHI [JP]) 11 February 2021 (2021-02-11) | 1-6, 8-14,17, 18 |
| Y | * paragraphs [0001], [0005], [0052], | 15,16 |
| A | [0054], [0063], [0065], [0132], [0139] * | 7 |
| | ----- | |
| Y | LOZANO-ANDRÉS ESTEFANÍA ET AL: "Intrinsic variability of fluorescence calibrators impacts the assignment of MESF or ERF values to nanoparticles and extracellular vesicles by flow cytometry", NANOMEDICINE: NANOTECHNOLOGY, BIOLOGY, AND MEDICINE ELSEVIER, AMSTERDAM, NL, vol. 56, 23 November 2023 (2023-11-23), XP087460742, ISSN: 1549-9634, DOI: 10.1016/J.NANO.2023.102720 [retrieved on 2023-11-23] * abstract * * page 2, left-hand column, paragraph 1 - paragraph 4 * * page 3, right-hand column, last paragraph - page 4 * | 15 |
| | ----- | |
| Y,D | JP 2023 521872 A (MASSACHUSETTS GEN HOSPITAL [US]) 25 May 2023 (2023-05-25) * abstract * * paragraph [0007] * | 16 |
| | ----- | |
| A | EP 3 699 588 A1 (KONICA MINOLTA INC [JP]) 26 August 2020 (2020-08-26) * paragraph [0108] * | 7 |
| | ----- | |

**CLASSIFICATION OF THE APPLICATION (IPC)**

INV.
G01N21/27
G01N21/64

**TECHNICAL FIELDS SEARCHED (IPC)**

G01N

The present search report has been drawn up for all claims

| Place of search | Date of completion of the search | Examiner |
|---|---|---|
| Munich | 14 April 2026 | D'Alessandro, Davide |

## ANNEX TO THE EUROPEAN SEARCH REPORT
## ON EUROPEAN PATENT APPLICATION NO.

EP 25 20 8877

14-04-2026

| Patent document cited in search report | Publication date | Patent family member(s) | | Publication date |
|---|---|---|---|---|
| US 2021041342 A1 | 11-02-2021 | US | 2021041342 A1 | 11-02-2021 |
| | | WO | 2019181205 A1 | 26-09-2019 |
| JP 2023521872 A | 25-05-2023 | CN | 115605742 A | 13-01-2023 |
| | | EP | 4136431 A1 | 22-02-2023 |
| | | JP | 2023521872 A | 25-05-2023 |
| | | US | 2023160809 A1 | 25-05-2023 |
| | | WO | 2021211756 A1 | 21-10-2021 |
| EP 3699588 A1 | 26-08-2020 | EP | 3699588 A1 | 26-08-2020 |
| | | JP | 7184046 B2 | 06-12-2022 |
| | | JP | WO2019078230 A1 | 17-12-2020 |
| | | US | 2021192786 A1 | 24-06-2021 |
| | | WO | 2019078230 A1 | 25-04-2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023521872 A

**Non-patent literature cited in the description**

- **JEONG, MI HO et al.** Plasmon-Enhanced Single Extracellular Vesicle Analysis for Cholangiocarcinoma Diagnosis. *Advanced Science*, January 2023, vol. 10 (8), 2205148